# EUROPÄISCHE PATENTSCHRIFT

(11) **EP 2 641 087 B1**
(45) Veröffentlichungstag und Bekanntmachung des Hinweises auf die Patenterteilung: **20.01.2016**
(21) Anmeldenummer: 12710164.0
(22) Anmeldetag: 22.02.2012
(51) Int. Cl.: G01N 33/543

(54) **MAGNETISCHE DURCHFLUSSZYTOMETRIE FÜR HOHEN PROBENDURCHSATZ**
MAGNETIC FLOW CYTOMETRY FOR HIGH SAMPLE THROUGHPUT
CYTOMÉTRIE DE FLUX MAGNÉTIQUE POUR HAUT DÉBIT DE PASSAGE D'ÉCHANTILLONS

(30) Priorität: 28.02.2011 DE 102011004806
(43) Veröffentlichungstag der Anmeldung: 25.09.2013
(73) Patentinhaber: Siemens Aktiengesellschaft, 80333 München (DE)
(72) Erfinder: HAYDEN, Oliver, 91074 Herzogenaurach (DE); HELOU, Michael, Johannes, 93051 Regensburg (DE); REISBECK, Mathias, 93083 Obertraubling (DE); TEDDE, Sandro, Francesco, 91058 Erlangen (DE)
(86) Internationale Anmeldenummer: PCT/EP2012/052977
(87) Internationale Veröffentlichungsnummer: WO 2012/116913

(56) Entgegenhaltungen:
- CARR C ET AL: "Magnetic Sensors for Bioassay: HTS SQUIDs or GMRs?", IEEE TRANSACTIONS ON APPLIED SUPERCONDUCTIVITY, IEEE SERVICE CENTER, LOS ALAMITOS, CA, US, Bd. 17, Nr. 2, 1. Juni 2007 (2007-06-01), Seiten 808-811, XP011188120, ISSN: 1051-8223, DOI: 10.1109/TASC.2007.897369
- INGLIS D W ET AL: "Continuous microfluidic immunomagnetic cell separation", APPLIED PHYSICS LETTERS, AIP, AMERICAN INSTITUTE OF PHYSICS, MELVILLE, NY, US, Bd. 85, Nr. 21, 22. November 2004 (2004-11-22), Seiten 5093-5095, XP002612323, ISSN: 0003-6951, DOI: 10.1063/1.1823015
- YANG SUNG-YI ET AL: "Micro flow cytometry utilizing a magnetic bead-based immunoassay for rapid virus detection", BIOSENSORS & BIOELECTRONICS, Bd. 24, Nr. 4, Dezember 2008 (2008-12), Seiten 855-862, XP002674649, ISSN: 0956-5663

## Beschreibung

Die vorliegende Erfindung betrifft die magnetische Zelldetektion im Durchfluss.

Im Bereich der Zellmessung und Zelldetektion sind neben optischen Messverfahren, wie Streulicht- oder Fluoreszenzmessung, auch magnetische Detektionsverfahren bekannt, bei denen die zu detektierende Zellsorte mittels magnetischer Labels markiert wird.

Insbesondere sind zur magnetbasierten Messung Verfahren bekannt, bei denen magnetisch markierte Zellen mittels Magnetophorese aus einer komplexen Zellsuspension, z.B. einer Blutprobe, aussortiert werden. Dazu müsste diese komplexe Suspension erst dementsprechend aufbereitet werden, dass zu detektierenden Zellen daraus abgetrennt werden können. Die magnetische Markierung erfolgt insbesondere dadurch, dass zellspezifische Marker in die komplexe Zellprobe eingeführt werden. Magnetophorese wird bisher zur Sortierung von magnetisch markierten Zellen oder allgemein magnetischen Partikeln verwendet.

Im Bereich der magnetoresistiven Sensorik für Zelldetektion ist es aber auch möglich, magnetisch markierte Zellen in einer komplexen Suspension im Durchfluss dynamisch zu zählen. Dazu ist es wichtig, dass die Zellen vereinzelt nacheinander über den Sensor fließen und dass die magnetisch markierten Zellen in ausreichender Nähe zum magnetoresistiven Sensor über diesen hinweggeführt werden.

In einem magnetischen Durchflusszytometer werden markierte Zellen in einem Kanal daher oberflächennah über einen Magnetsensor transportiert. Die Nähe einer magnetisch markierten Zelle zum Sensor ist entscheidend, da das magnetische Streufeld der magnetischen Marker, anhand dessen die markierte Zelle letztlich durch den Sensor detektiert wird, mit der dritten Potenz zum Abstand abfällt.

Um sicherzustellen, dass eine markierte Zelle den Sensor in unmittelbarer Nähe passiert, ist es grundsätzlich denkbar, den Durchmesser des Kanals, durch den die Zellprobe strömt, möglichst klein zu gestalten. D.h. im Extremfall ist der Kanaldurchmesser gerade so groß, dass einzelne Zellen passieren können. Problematisch wirkt sich hierbei natürlich aus, dass die Anwesenheit von Verunreinigungen bzw. störenden Partikeln sehr schnell zu einer Verstopfung des Kanals führt.

Wird der Kanal dagegen größer ausgelegt, steigt auch die Wahrscheinlichkeit, dass einige markierte Zellen den Sensor außerhalb von dessen Reichweite passieren und somit nicht detektiert werden. Dem kann dadurch entgegen gewirkt werden, dass die magnetisch markierten Zellen am Sensor angereichert werden: Es hat sich gezeigt, dass sich eine möglichst lange Anreicherungsstrecke durch einen Mikrofluidikkanal von bis zu 1 cm Länge positiv darauf auswirkt, dass am Ende der Anreicherungsstrecke nahezu 100 % der magnetisch markierten Zellen aus der komplexen Suspension so am Kanalboden angereichert sind, dass eine Erfassung durch einen Magnetsensor möglich ist. Eine derart lange Anreicherungsstrecke auf einem Halbleitersubstrat, auf welchem das magnetoresistive Bauteil ausgebildet ist anzuordnen, führt jedoch zu einem hohen Aspektverhältnis des Substrats, welches neben hohen Kosten für die Gesamtfläche des Halbleitersubstrats, insbesondere für Silizium Dies, auch zu Problemen bei der Prozessierung im Herstellungsprozess führt. Je höher die Geschwindigkeit des Durchflusses und je höher die Zellkonzentration in der Probe, desto länger muss die Ausrichtungsstrecke gewählt werden, um zu einer ausreichend ausreichenden Anreicherung der magnetisch markierten Zellen zum Zeitpunkt des Sensorübertritts zu gewährleisten.

Der Artikel "Magnetic Sensors for Bioassay: HTS SQUIDs or GMRs?"; C. Carr et al.; IEEE Transactions on applied Superconductivity, Vol. 17, Nr. 2, S. 808-811 betrifft Sensoren zur Erfassung magnetischer Nanopartikel. Als magnetische Sensoren werden SQUID-Sensoren und GMR-Sensoren genannt.

In dem Artikel "Continuous microfluidic immunomagnetic cell separation"; D. Inglis et al.; Applied Physics Letters, vol. 85, Nr. 21, S. 5093-5095 ist eine Trennung von magnetisch markierten Zellen aus einem Volumenstrom betrieben. Hierzu wird der Volumenstrom über ein Siliziumsubstrat mit integrierten magnetischen Streifen geleitet.

Der Bericht "Micro flow cytometry utilizing a magnetic beadbased immunoassay for rapid virus detection"; Sung-Yi Yang et al.; Biosensors and Bioelectronics 24 (2008), S. 855-862 beschreibt ein Verfahren zur Detektion von Viren durch Anbindung der Viren an magnetische "beads". Die Viren werden anschließend mittels Mikroflusszytometrie detektiert.

Es ist Aufgabe der vorliegenden Erfindung, eine Vorrichtung zur magnetischen Zelldetektion zu schaffen, die eine Verkleinerung des Halbleiter-Chips bei Erhöhung des Probendurchsatzvolumens ermöglicht.

Die Aufgabe wird durch eine Vorrichtung zur magnetischen Durchflusszytometrie gemäß Patentanspruch 1 gelöst. Ein dazugehöriges Herstellungsverfahren wird in Patentanspruch 10 angegeben. Ein Verfahren zur magnetischen Zelldetektion wird in Patentanspruch 15 beschrieben. Vorteilhafte Ausgestaltungen der Erfindung sind Gegenstand der Unteransprüche.

Die erfindungsgemäße Vorrichtung zur magnetischen Durchflusszytometrie umfasst einen magnetoresistiven Sensor auf einem Substrat und eine Anreicherungsstrecke. Dabei ist die Anreicherungsstrecke in einen ersten und zweiten Abschnitt aufgeteilt. Der zweite Abschnitt der Anreicherungsstrecke ist auf dem Substrat angeordnet, der erste Abschnitt der Anreicherungsstrecke ist neben dem Substrat auf einem Träger angeordnet, so dass die Anreicherungsstrecke über einen Rand des Substrats hinweg verläuft.

Um eine möglichst lange Anreicherungsstrecke auszubilden, ohne dafür das Halbleitersubstrat, auf dem der magnetoresistive Sensor aufgebaut ist, unnötig zu vergrößern, wird die Anreicherungsstrecke neben dem Substrat ausgebildet. Insbesondere teilen sich Anreicherungsstrecke und Substrat einen gemeinsamen Träger, z.B. eine Leiterplatine. Auf diese Leiterplatine wird das Halbleitersubstrat mit dem Sensor aufgebracht, elektrisch angebunden und so in ein Package eingebracht, das die elektrischen Kontakte isoliert und vor Korrosion sowie vor mechanischer Beschädigung geschützt sind. Auf diesem Trägersubstrat, beziehungsweise auf dem Packaging-Material, das auf das Trägersubstrat aufgebracht ist, kann die Anreicherungsstrecke nun beliebig lang ausgeführt werden. Beispielsweise kann die Anreicherungsstrecke mäanderförmig ausgestaltet sein und in mehreren Bahnen die über Krümmungen verbunden sind, verlaufen, bis sie den Halbleiterchip mit dem Sensor erreicht. Insbesondere wird für die Ausbildung eine Durchflusskammer, durch die eine Zellprobe fließen kann, das Packaging-Material verwendet. Das Packaging erfolgt insbesondere in einem Spritzgussverfahren, durch das eine Durchflusskammer hergestellt werden kann.

Vorteilhafterweise umfasst die Anreicherungsstrecke magnetische Führungsstreifen, die insbesondere ferromagnetisch sind. Als ferromagnetisches Material für die Führungsstreifen kommt beispielsweise Nickel in Frage. Auch ferromagnetische Legierungen sind dazu einsetzbar.

In einer vorteilhaften Ausgestaltung der Erfindung ist die Durchflusskammer, insbesondere ein Mikrofluidikkanal, so entlang der Anreicherungsstrecke ausgebildet, dass eine durch den Mikrofluidikkanal strömende magnetisch markierte Zellprobe an den magnetischen Führungsstreifen der Anreicherungsstrecke ausgerichtet wird. D.h. die magnetischen Führungsstreifen und die magnetisch markierten Zellen wechselwirken so, dass die Zellen eine Ausrichtung innerhalb der Zellsuspension erfahren, so dass das Streufeld ihrer magnetischen Labels zu einem möglichst hohen Signal über dem Sensor führt.

In einer weiteren vorteilhaften Ausgestaltung der Erfindung umfasst die Vorrichtung einen Magneten, der so angeordnet ist, dass eine durch den Mikrofluidikkanal strömende magnetisch markierte Zellprobe durch das Magnetfeld dieses Magneten am Kanalboden angereichert wird. D.h., dass zusätzlich zur Führung durch die magnetischen Führungsstreifen das Magnetfeld eines Magneten, insbesondere eines Permanentmagneten, auf die markieren Zellen innerhalb der Zellsuspension eine magnetische Kraft ausübt und diese in eine Richtung aus der Zellsuspension zum Kanalboden hin bewegt.

Diese Anreicherung und Ausrichtung der magnetisch markierten Zellen hat den Vorteil, dass die Konzentration der magnetisch markierten Zellen nahe am Kanalboden und so nahe am magnetoresistiven Sensor erhöht wird. Der Sensor ist zweckdienlicherweise so am Kanalboden angebracht, dass dieser im Wesentlichen alle markierten Zellen detektieren kann. Sind die Zellen vor der Anreicherung und Ausrichtung noch im gesamten Probenvolumen gleich verteilt, so führt die Anreicherung und Ausrichtung dazu, dass eine Einzelzelldetektion am Sensor gewährleistet wird.

In einer vorteilhaften Ausgestaltung der Erfindung sind ein erster Abschnitt und ein zweiter Abschnitt des Mikrofluidikkanals sowie der magnetoresistive Sensor so angeordnet, das eine durch den Mikrofluidikkanal strömende magnetisch markierte Zellprobe zunächst durch den ersten Abschnitt des Mikrofluidikkanals über den ersten Abschnitt der Anreicherungsstrecke und dann durch den zweiten Abschnitt des Mikrofluidikkanals über den zweiten Abschnitt der Anreicherungsstrecke und über den Sensor geführt wird. Dabei sind die jeweils zweiten Abschnitte von Mikrofluidikkanal und Anreicherungsstrecke so ausgestaltet, dass diese einen Versatz zu den jeweils ersten Abschnitten von Mikrofluidikkanal und Anreicherungsstrecke korrigieren können. Ein derartiger Versatz kann bei der Zusammenführung von dem Substrat, auf dem der magnetoresistive Sensor angeordnet ist und dem Träger, auf dem wiederum das Substrat angeordnet wird, zustande kommen. Die jeweils zweiten Abschnitte von Mikrofluidikkanal und Anreicherungsstrecke bewirken, dass zumindest im Bezug auf die Anreicherung und Ausrichtung der strömenden magnetisch markierten Zellprobe, dieser Versatz korrigiert wird. D.h., das die am Sensor ankommenden magnetisch markierten Zellen so am Kanalboden angereichert und so ausgerichtet sind, als ob kein Versatz innerhalb der Anreicherungsstrecke durch den Übergang vom Träger auf das Substrat vorhanden wäre.

Insbesondere hat die Anreicherungsstrecke eine Mindestlänge von 15000 µm. Dabei aber hat das Substrat insbesondere eine größte Ausdehnung von höchstens 18000 µm. Da insbesondere Halbleitersubstrate wie Silizium sehr hohe Kosten haben, ist der geringe Flächenbedarf von großem Vorteil. Die geringe benötigte Fläche an Halbleitersubstrat wird dadurch gewährleistet, dass nur ein gerade so langer Anreicherungsstreckenabschnitt auf dem Substrat vorhanden sein muss, das ein Versatz bei der Montage von Substrat auf Träger ausgeglichen wird. Der Hauptteil der Anreicherung und Ausrichtung kann jedoch in dem ersten Abschnitt der Anreicherungsstrecke auf dem Träger passieren.

Es hat sich gezeigt, dass die Mindestlänge der Anreicherungsstrecke den Vorteil hat, dass auch hochkonzentrierte Zellproben am Ende der Anreicherungsstrecke so am Kanalboden angereichert und so entlang der magnetischen Führungslinien der Anreicherungsstrecke ausgerichtet werden können, dass zum Zeitpunkt des Überstreichen des magnetoresistiven Sensors eine Einzelzellerkennung gewährleistet ist.

Bei der Zusammenführung von Halbleiterchip und Träger kann es zu einem geringen Offset, einem Versatz kommen, der typischerweise geringer als 100 µm ist. D.h. der erste Teil der Anreicherungsstrecke ist auf einen anderen Punkt ausgerichtet als dann tatsächlich der magnetoresistive Sensor angeordnet ist. Um dies auszugleichen, ist auch auf dem Halbleiterchip, auf dem letzten Teil des Mikrofluidikkanals zum Sensor, noch eine Anreicherungsstrecke ausgebildet, welche ferromagnetische Streifen aufweist, durch die die angereicherten und ausgerichteten Zellen auf den Sensor hin konzentriert werden. Die Zellen folgen insbesondere dem laminaren Strömungsprofil. Für die Anreicherung der Zellen am Kanalboden, möglichst nahe an der Sensoroberfläche, werden die magnetischen Zellen in einem magnetischen Gradientenfeld ausgesetzt, welches z.B. durch einen Permanentmagneten unterhalb der Bauteilanordnung erzeugt wird, oder durch zwei Magnete ober- und unterhalb des Durchflusszytometers.

Der magnetoresistive Sensor der Vorrichtung ist insbesondere ein GMR-Sensor (GMR = Giant Magneto Resistance bzw. Riesenmagnetowiderstand), Beispielsweise ist der magnetoresistive Sensor der Vorrichtung ein TMR-Sensor (TMR = Tunnel Magneto Resistance bzw. Tunnelmagnetowiderstand) oder der magnetoresistive Sensor der Vorrichtung ist ein AMR-Sensor (AMR = Anisotroper Magnetowiderstand).

Die Herstellung einer oben beschriebenen Vorrichtung umfasst die Schritte der Herstellung des magnetoresistiven Sensors auf einem Halbleitersubstrat, die Aufbringung des zweiten Abschnitts der Anreicherungsstrecke auf das Halbleitersubstrat, das Packaging des Halbleitersubstrats auf einen Träger sowie das Herausführen der elektrischen Kontakte des magnetoresistiven Sensors auf den Träger und schließlich die Ausformung eines ersten Abschnitts der Anreicherungsstrecke. Dieses Verfahren hat den Vorteil, dass eine geringe Halbleitersubstratfläche so genutzt wird, dass eine große Anreicherungsstrecke aufgebaut werden kann. Das Packaging des Halbleiterchips ist ein gängiges Verfahren der Mikrosystemtechnik, das zur Isolation, zum Korrosionsschutz sowie zum Schutz gegen Beschädigung der Kontakte und zur Befestigung des Halbleiterchips auf einem Träger z.B. einer Leiterplatte dient. Das Packaging und der Träger werden beispielsweise weiter so genutzt, dass auf dem Träger ein langer Abschnitt der Anreicherungsstrecke so ausgeformt wird, dass darauf die hauptsächliche Anreicherung und Ausrichtung erfolgt bevor die Zellsuspension auf den Halbleiterchip mit dem Sensor geleitet wird.

Der Vorteil liegt insbesondere darin, dass so für niedrig konzentrierte Proben von etwa 1 Zelle pro µl große Anreicherungsstrecken mit großem Volumendurchsatz realisiert werden können, bei niedrigem Silizium-Verbrauch. Der Silizium-Die Footprint wird demnach so weit wie möglich reduziert. Als "Die" wird beispielsweise der ungehäuste Halbleiter-Chip, ein integriertes elektronisches Bauelement, das Halbleiter- oder Sensor-Substrat bezeichnet. Nach der Fertigstellung der integrierten Sensor-Schaltung auf dem Halbleiterchip, wird diese in einem "Package" verkapselt, um sie gegen Beschädigung oder Korrosion zu schützen. Dazu wird der Halbleiterchip zunächst auf ein Trägersubstrat aufgebracht und es werden die elektrischen Kontakte der integrierten Schaltung auf das Trägersubstrat herausgeführt. Dies geschieht beispielsweise durch Drahtbonden oder mittels Durchkontaktierungen. Als Material für das Packaging werden beispielsweise Keramiken oder Polymere, wie Epoxide verwendet. Das Packaging ist demnach ein notwendiger Verfahrensschritt um ein Bauteil zu schaffen, das Umwelteinflüssen ausgesetzt werden kann. Dieses Beispiel für eine Ausführungsform der Erfindung bietet nun den großen Vorteil dieses Packaging doppelt zu nutzen, indem zum einen die zusätzliche Fläche für die Anordnung der Anreicherungsstrecke darauf ausgenutzt wird, zum anderen das Packaging-Material selbst zur Ausbildung der Durchflusskammer dient und dies insbesondere in einem einzelnen Verfahrensschritt passiert.

In einer vorteilhaften Ausgestaltung der Erfindung wird in dem Verfahren in dem Packaging-Schritt des Halbleitersubstrats aus dem Packaging-Material ein Mikrofluidikkanal ausgeformt. Der Packaging-Schritt kann insbesondere mittels Spritzgießen erfolgen. Damit kann mittels Spritzgießtechnik ein Mikrofluidikkanal ausgeformt werden.

In einer vorteilhaften Ausgestaltung der Erfindung werden die magnetischen Führungsstreifen der Anreicherungsstrecke insbesondere des ersten Abschnitts der Anreicherungsstrecke direkt auf den Kanalboden abgeschieden. Dazu werden beispielsweise Verfahren wie thermisches Verdampfen oder Sputtern verwendet. Durch die Ausgestaltung des Mikrofluidikkanals auf das Packaging-Material wird also die magnetische Führung durch die Führungsstreifen innerhalb des Kanals angeordnet.

Für den Teil der Anreicherungsstrecke, der auf dem Halbleitersubstrat angeordnet ist, können die magnetischen Führungsstreifen wiederum direkt auf das Halbleitersubstrat abgeschieden werden. Dazu können wiederum thermisches Verdampfen oder ein Sputter-Prozess dienen.

Zu einem Verfahren zur magnetischen Zelldetektion wird eine magnetisch markierte Zellprobe in eine oben beschriebene Vorrichtung mit Anreicherungsstrecke auf einem zusätzlichen Trägersubstrat neben dem Halbleitersubstrat injiziert.

Die Anreicherung über ein externes Feld, z.B. das Feld eines Permanentmagneten, und die magnetophoretische Ausrichtung mittels der ferromagnetischen Führungsbahnen erfolgt bevorzugt in-situ während des Messvorgangs. Daher ist eine ausreichend lange Ausrichtungsstrecke für die magnetisch markierten Zellen erforderlich, um eine gewünschte Wiederfindungsrate von den markierten Zellen von im Wesentlichen 100 % zu gewährleisten. Einflussfaktoren auf die genaue erforderliche Länge der Anreicherungs- und Ausrichtungsstrecke mit den ferromagnetischen Bahnen sind
1. die Geschwindigkeit mit der die Zellprobe durch den Mikrofluidikkanal gepumpt wird,
2. die Magnetfeldstärke des angelegten Anreicherungsmagnetfelds,
3. die Konzentration der superparamagnetisch markierten Zellen in der Suspension, sowie
4. die magnetischen Eigenschaften der eingesetzten Marker,
5. die Zusammensetzung und rehologischen Eigenschaften der Zellsuspension, d.h. z.B. deren Fließeigenschaft und
6. die Sorte der markierten Zellen und deren Isotopenanzahl auf der Zelloberfläche und damit die Anzahl paramagnetischer Marker pro Zelle, die Stärke des zu detektierenden Streufelds bestimmt.

Die Zellsuspension wird insbesondere mittels eines Druckgefälles durch den Mikrofluidikkanal gepumpt. Das Druckgefälle kann beispielsweise durch manuelle Bedienung einer Spritze oder eines Spritzensystems erzeugt werden. Dadurch ist gewährleistet, dass sich eine laminare Strömung der Zellprobe ohne Rezirkulation einstellt. Da die Zellen und das die Zellen umgebende komplexe Medium näherungsweise die gleiche Dichte aufweisen, tritt auch in den Krümmungsbereichen des mäanderförmigen Fluidikkanals nur eine geringe Fliehkraft auf und die markierten Zellen können auf ihrer Bahn verbleiben.

Bei der magnetischen Durchflusszytometrie ist also entscheidend, dass die magnetisch markierten Zellen sehr nah am magnetoresistiven Sensor vorbeitransportiert werden. Da die Zellprobe durch eine Durchflusskammer, z.B. einem Mikrofluidikkanal fließt, müssen die markierten Zellen in dieser Durchflusskammer nahe an deren Innenfläche transportiert werden, wo der magnetoresistive Sensor angebracht ist. Insbesondere ist die Kanalwand in direktem Kontakt über dem Magnetsensor angebracht. In alternativen Ausführungsformen ist der magnetoresistive Sensor in die Kanalwand eingebettet. Als magnetische Markierung dienen bevorzugt superparamagnetische Labels. Als magnetoresistive Sensoren kommen GMR-, TMR- oder AMR-Sensoren in Frage. Die Nähe der magnetisch markierten Zelle zum Sensor ist deshalb so entscheidend, da das magnetische Streufeld der magnetischen Markierung im Nahfeldbereich mit dritter Potenz zum Abstand abfällt. Zusätzlich zur Anreicherung der magnetisch markierten Zellen an der Sensoroberfläche wirkt sich eine Ausrichtung der magnetisch markierten Zellen positiv auf deren Detektierbarkeit aus. Dabei werden die magnetisch markierten Zellen vorzugsweise so in Durchflussrichtung ausgerichtet, dass das Magnetfeld der magnetischen Markierung ein möglichst deutliches Signal im Sensor hervorruft. Bei der magnetischen Durchflusszytometrie ist eine möglichst genaue Differenzierung zwischen falschpositiven und positiven Signalen erforderlich. Dazu muss für positive Signale ein möglichst hoher Schwellwert für das Signal gesetzt werden können um sie von Rauschsignalen zu unterscheiden.

Im Gegensatz zur Methode, die magnetisch markierten Zellen vereinzelt über einen Sensor zu führen, indem sie in einem Mikrofluidikkanal durch dessen Durchmesser so eingeengt werden, dass nur einzelne Zellen diesen passieren können, weist das Verfahren den Vorteil auf, eine im Wesentlichen 100%ige Einzelzelldetektion zu ermöglichen, direkt aus der unvorbereiteten komplexen Suspension heraus. Somit ist der große Nachteil der sozusagen mechanischen Vereinzelung der Zellen überwunden, dass diese zu Verstopfungen des Fluidiksystems führt. Auch könnten in einer derartigen Messvorrichtung keine magnetisch markierten Zellen von unterschiedlichem Durchmesser exakt einzeln bestimmt werden. Die Zellen haben beispielsweise Durchmesser im Bereich von ca. 3 bis 30 µm. Bevorzugt werden sie durch einen sehr viel breiteren Mikrofluidikkanal geführt, der im Durchmesser das 10- bis 1000fach beträgt. Der Sensor oder eine Sensor-Array ist dabei quer zur Flussrichtung angeordnet und beträgt beispielsweise 30 µm Breite entsprechend dem Zelldurchmesser.

### Kurze Beschreibung der Zeichnung

Ausführungsformen der vorliegenden Erfindung werden in exemplarischer Weise mit Bezug auf die Figuren 1 bis 6 der angehängten Zeichnung beschrieben:
- Figur 1: zeigt einen Querschnitt durch Mikrofluidikkanal und Substrat,
- Figur 2: zeigt die dazugehörige Draufsicht mit Magnetlinien,
- Figur 3: zeigt wiederum einen Querschnitt durch Mikrofluidikkanal und Substrat und
- Figur 4: die dazugehörige Draufsicht mit den Magnetlinien.
- Figur 5: zeigt einen weiteren Querschnitt durch Mikrofluidikkanal und Substrat und
- Figur 6: zeigt einen weiteren Querschnitt durch Mikrofluidikkanal und Substrat,
- Figur 7: zeigt eine mäanderförmige Anreicherungsstrecke,
- Figur 8: zeigt die magnetischen Führungslinien in der ersten Krümmung der Anreicherungsstrecke.

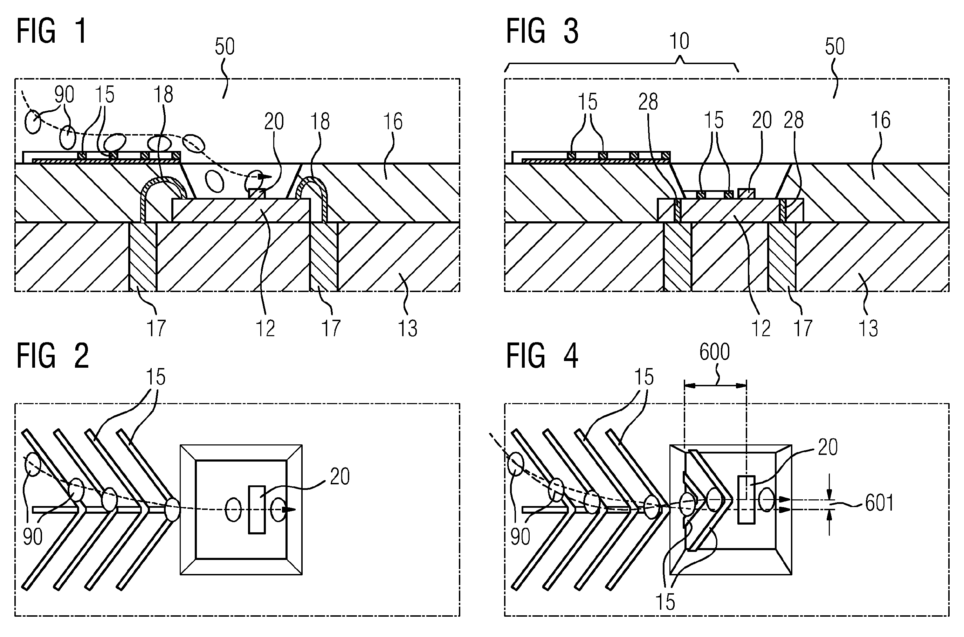

Die Figur 1 zeigt einen Querschnitt durch eine Ausführungsform der Messvorrichtung und die Figur 2 die dazugehörige Draufsicht. Die Figur 3 zeigt ein alternatives Ausführungsbeispiel der Erfindung im Querschnitt und die Figur 4 die dazugehörige Draufsicht.

Der Halbleiterchip 12, der als Substrat für den Messsensor 20 dient, ist auf eine Trägerplatte 13 aufgebracht. Die Trägerplatte 13 ist beispielsweise eine Leiterplatte für elektronische Bauteile, insbesondere eine Kupferleiterplatte. Diese Leiterplatte 13 weist Kontakte 17 auf, die beispielsweise, wie in den Figuren 1a und 2a gezeigt, Durchführungen durch die Trägerplatte 13 darstellen. D.h. sie verbinden die Oberseite und Rückseite der Trägerplatte 13 elektrisch. An diese Durchkontaktierungen oder Kontakte 17 werden die Kontakte des Messsensors 20 auf dem Halbleiterchip 12 durch Drahtbonden und dementsprechend elektrische Drahtverbindungen 18 verbunden wie in Figur 1 gezeigt oder alternativ wie in Figur 3 gezeigt mit sogenannten Through silicon vias 28 elektrisch mit den Kontakten 17 der Trägerplatte 13 verbunden.

Über einen Teil des Halbleiterchips 12, insbesondere den Teil mit den elektrischen Kontakten 18, 28 wird das Packaging-Material 16 abgeschieden und verbindet somit den Halbleiterchip 12 mit der Trägerplatine 13. Die Kontakte 17, 18, 28 sind über das Packaging-Material 16 elektrisch isoliert sowie vor Korrosion oder mechanischer Beschädigung geschützt. Der Teil des Halbleiterchips 12 der vom Packaging-Material 16 frei bleibt, weist den magnetoresistiven Sensor 20 auf. Über das Packaging-Material 16 hinweg kann nun eine Zellprobe 90 auf diesen freigelassenen Bereich des Halbleiterchips mit dem Sensor 20 fließen. Dazu ist die Anreicherungsstrecke 10 über den Rand des Substrats 12 hinweg angeordnet. Wie durch die magnetisch markierten Zellen 90 und die gestrichelten Pfeile angedeutet, wird eine Strömung der Zellprobe 90 über den Sensor 20 hinweg erzeugt. Dazu ist insbesondere ober- oder unterhalb der Messvorrichtung ein Permanentmagnet angeordnet, in dessen Magnetfeld die markierten Zellen 90 auf dem Packaging-Material 16 und auf dem Halbleiterchip 12 angereichert werden, d.h. in dem Magnetfeld an das Packaging-Material 16 und den Halbleiterchip 12 hin geführt werden. Neben der Anreicherung der Zellen 90 an der Sensoroberfläche werden die Zellen 90 zusätzlich entlang magnetischer Führungslinien 15 ausgerichtet, die in den Figuren 1a bis 2b auch jeweils im Querschnitt und in der Draufsicht zu sehen sind. Im Querschnitt sieht man nur, dass die einzelnen magnetischen Führungslinien 15 auf eine Mittenspur der Anreicherungsstrecke 10 in Richtung des Magnetsensors 20 führen. In der Draufsicht in den Figuren 1b und 2b ist die bevorzugte Anordnungsgestaltung der Magnetführungslinien 15 gezeigt, die wie ein Fischgrätenmuster aussieht. Die magnetischen Führungslinien 15 weisen in einem Winkel von weniger als 90° auf die Mittenlinie der Anreicherungsstrecke 10 hin und führen somit die magnetisch markierten Zellen 90 vom Rand der Anreicherungsstrecke 10 hin zum zentralen Weg der Anreicherungsstrecke 10, so dass diese zentral über den magnetoresistiven Widerstand 20 geführt werden können.

Der Großteil der Anreicherungsstrecke 10, die über den Rand des Substrats 12 hinausragt, liegt auf dem Packaging-Material 16, welches beliebig großflächig abgeschieden werden kann. D.h. die Grundträgerplatte 13 bestimmt die Gesamtgröße der Messvorrichtung. Darauf sind sehr einfach und kostengünstig große Anreicherungsstrecken 10 realisierbar. Erreichen die magnetisch markierten Zellen 90 den Halbleiterchip 12 mit dem Sensor 20, sind diese schon an der Sensoroberfläche angereichert und entsprechend ausgerichtet. Die kurze Strecke auf dem Halbleiterchip 12 vor dem Sensor 20 weist aber wiederum eine kurze Anreicherungsstrecke 600 auf, die dazu dient, dass ein eventueller Versatz 601 der Anreicherungsstrecke auf dem Packaging-Material 16 zum Sensor 20 hin ausgeglichen werden kann. Ein derartiger Versatz 601, wie in der Figur 4 markiert, kann bei der Montage des Halbleiterchips 12 auf dem Träger 13 passieren. Geringe Offsets 601 können jedoch durch eine kurze Anreicherungsstrecke 600 ausgeglichen werden, ohne dass eine weitere lange Anreicherungsstrecke 10 nötig ist. Somit reichen kurze Anreicherungsstrecken 600 auf dem Halbleiterchip 12 aus, die dessen Gesamtgröße nicht wesentlich erhöhen, die magnetisch markierten Zellen 90 jedoch zentral über den Sensor 20 führen.

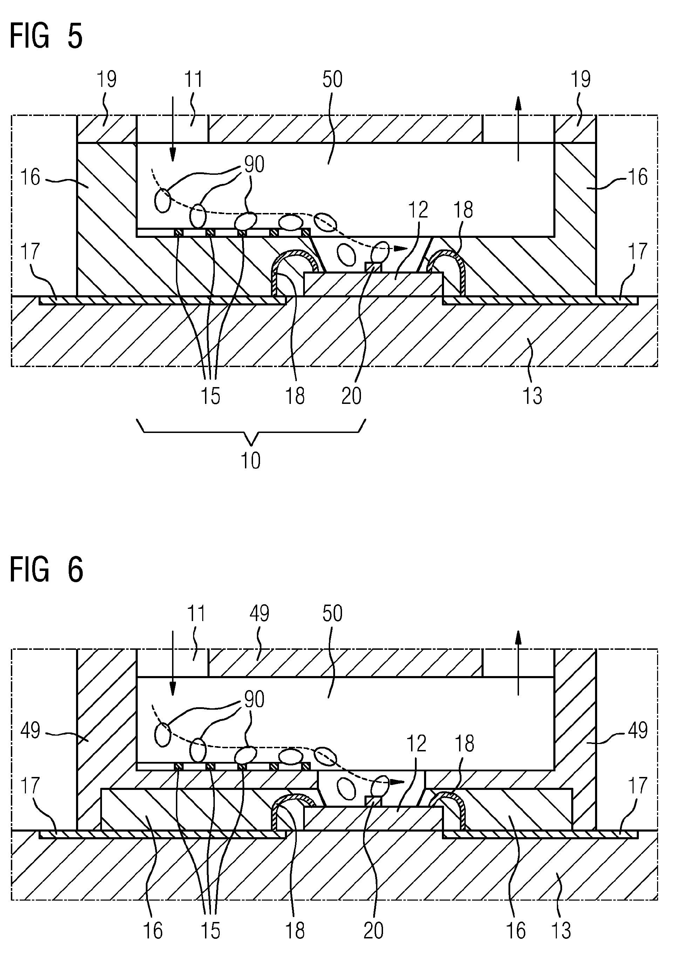

In Figur 5 ist eine mögliche Ausführungsform des Mikrofluidikkanals 50 auf einer Messvorrichtung gezeigt. Wiederum zeigt die Figur 5 ein Trägersubstrat 13 auf das der Halbleiterchip 12 mittels Drahtbonden 18 kontaktiert wird. Auch ist wiederum das Packaging 16 gezeigt, welches die elektrischen Kontakte 17, 18 isoliert und schützt. Des Weiteren ist der Querschnitt durch die magnetischen Führungslinien 15 gezeigt, entlang derer die magnetisch markierten Zellen 90 geführt werden. Die Oberfläche von Packaging-Material 16 und Halbleiterchip 12 stellt sozusagen den Boden des Mikrofluidikkanals 50 dar, an dem die magnetisch markierten Zellen 90 angereichert werden. Das Packaging-Material 16 wird insbesondere in einem Spritzgussverfahren so aufgetragen, dass ein Mikrofluidikkanal 50 entsteht, durch den die Zellprobe 90 geleitet werden kann. Insbesondere weist dieser Kanal 50 Ein- und Auslässe 11 für die Zellprobe 90 auf, die in der Figur 5 durch ein- und ausweisende Pfeile gekennzeichnet sind. Die Figur 5 zeigt ein Beispiel in dem die Kanalwand durch das Packaging-Material 16 realisiert ist und in dem die Messvorrichtung, bzw. der Mikrofluidikkanal 50, durch eine Verkapselung 19 von oben her verschlossen wird.

Die Figur 6 zeigt wiederum einen Querschnitt durch eine alternative Ausführungsform. Im Unterschied zur Figur 5 ist hierfür nicht das Packaging-Material 16 als Kanalwand ausgestaltet, sondern nach dem Packaging-Schritt, der die elektrischen Kontakte 17, 18 verschließt, wird auf die Trägerplatte 13 und über das Packaging-Material 16 ein weiteres durch Spritzguss zu verarbeitendes Material 49 abgeschieden, aus dem der Mikrofluidikkanal 50 ausgebildet wird.

Der Querschnitt zeigt, dass der Mikrofluidikkanal 50 wieder von oben her geschlossen ist und lediglich einen Ein- und einen Auslass 11 aufweist, der durch Pfeile gekennzeichnet ist. Auch ist gezeigt, dass die magnetisch markierten Zellen 90 wiederum am Kanalboden, d.h. auf dem Substrat 12, insbesondere dem magnetischen Führungslinien 15 und anschließend auf dem Halbleiterchip 12 und dem Sensor 20 angereichert sind.

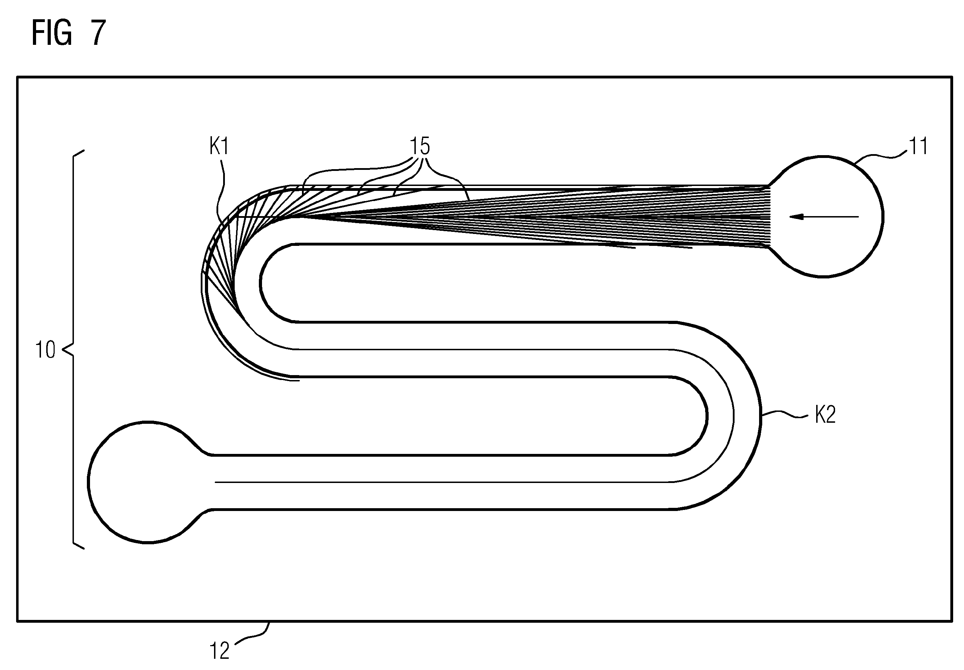

Die Figur 7 zeigt eine Draufsicht auf eine mäanderförmige Anreicherungsstrecke 10. Die Anreicherungsstrecke 10 weist drei gerade Teilstrecken auf, die über zwei Krümmungen K1, K2 miteinander verbunden sind. Die Anreicherungsstrecke 10 ist zum einen zur Ausrichtung aber auch zur Anreicherung von magnetisch markierten Zellen 90 am Kanalboden ausgestaltet. D.h. die Figur 7 zeigt einen Mikrofluidikkanal 50, der so entlang der Anreicherungsstrecke 10 angebracht ist, dass eine Zellprobe 90, die durch diesen Mikrofluidikkanal 50 geleitet wird, die magnetischen Kräfte eines Permanentmagneten zur Anreicherung am Kanalboden sowie auch die magnetische Wechselwirkung mit den magnetischen Führungslinien 15 erfährt. Die in Figur 7 gezeigten magnetischen Führungslinien 15 laufen entlang der Anreicherungsstrecke 10 direkt auf dem Substrat 12, welches insbesondere die Oberfläche eines Halbleiterchips ist. Entlang der ersten geraden Teilstrecke laufen die magnetischen Führungslinien 15 in einem spitzen Winkel auf eine Mittellinie der Anreicherungsstrecke 10 zu und führen somit die magnetisch markierten Zellen 90 in die Kanalmitte. Entlang der ersten Krümmung K1 laufen die magnetischen Führungslinien 15 von dem Rand der Anreicherungsstrecke 10, d.h. auch vom Rand des Mikrofluidikkanals 50 zur Mitte der Anreicherungsstrecke 10 hin. In diesem Beispiel ist eine zentrale magnetische Führungslinie gezeigt, die stets entlang der Kanalmitte angeordnet ist. Des Weiteren zeigt die Figur 7 in der Draufsicht auf die Anreicherungsstrecke 10, einen Einlass 11 für eine Zellprobe in den Mikrofluidikkanal.

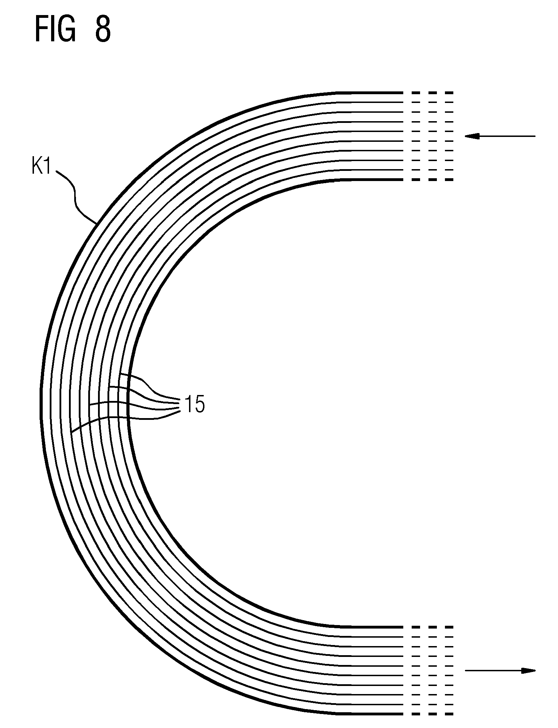

In der Figur 8 ist ein Ausschnitt der Anreicherungsstrecke 10 gezeigt mit der ersten Krümmung der Anreicherungsstrecke K1. In der Figur 8 ist eine alternative Ausführungsform der magnetischen Führungslinien 15 gezeigt. Diese können anstatt fächerförmig auf die Mittenlinie zuzulaufen auch halbkreisförmige Linien unterschiedlicher Radien sein, die jeweils in einem festen Abstand zu den Kanalwänden des Mikrofluidikkanals 50 eine Bahn beschreiben. In diesem Beispiel werden die magnetisch markierten Zellen 90 in der Zellprobe auf diesen Bahnen durch die Krümmung K1 geleitet. Die Pfeile deuten die Strömungsrichtung der Zellprobe durch die Krümmung K1 der Anreicherungsstrecke 10 an.

## Patentansprüche

1. Vorrichtung zur magnetischen Durchflusszytometrie mit
- einem magnetoresistiven Sensor (20) auf einem Substrat (12) und
- einer Anreicherungsstrecke (10) zur Ausrichtung und Anreicherung magnetisch markierter Zellproben (90), wobei
die Anreicherungsstrecke (10) einen ersten und einen zweiten Abschnitt aufweist, wobei der zweite Abschnitt auf dem Substrat (12) angeordnet ist und der erste Abschnitt neben dem Substrat (12) auf einem Träger (13) angeordnet ist, so dass die Anreicherungsstrecke (10) über einen Rand des Substrats (12) hinweg verläuft.

2. Vorrichtung nach Anspruch 1, wobei die Anreicherungsstrecke (10) magnetische Führungsstreifen (15) aufweist.

3. Vorrichtung nach Anspruch 2, wobei die magnetischen Führungsstreifen (15) ferromagnetisch sind.

4. Vorrichtung nach einem der vorstehenden Ansprüche 2 oder 3, wobei eine Durchflusskammer (50), insbesondere ein Mikrofluidikkanal, so entlang der Anreicherungsstrecke (10) ausgebildet ist, dass eine durch den Mikrofluidikkanal (50) strömende magnetisch markierter Zellproben (90) an den magnetischen Führungsstreifen (15) ausgerichtet wird.

5. Vorrichtung nach einem der vorstehenden Ansprüche mit einem Magneten, wobei der Magnet so angeordnet ist, dass eine durch den Mikrofluidikkanal (50) strömende magnetisch markierte Zellprobe (90) durch das Magnetfeld des Magneten am Kanalboden angereichert wird.

6. Vorrichtung nach einem der vorstehenden Ansprüche, wobei ein erster Abschnitt und ein zweiter Abschnitt des Mikrofluidikkanals (50) sowie der magnetoresistive Sensor (20) so angeordnet sind, dass eine durch den Mikrofluidikkanal (50) strömende magnetisch markierte Zellprobe (90) zunächst durch den ersten Abschnitt des Mikrofluidikkanals (50) über den ersten Abschnitt der Anreicherungsstrecke (10), dann durch den zweiten Abschnitt des Mikrofluidikkanals (50) über den zweiten Abschnitt der Anreicherungsstrecke (10) und über den Sensor (20) geführt wird, wobei die zweiten Abschnitte von Mikrofluidikkanal (50) und Anreicherungsstrecke (10) so ausgestaltet sind, dass durch diese ein Versatz zu den jeweils ersten Abschnitten von Mikrofluidikkanal (50) und Anreicherungsstrecke (10) in Bezug auf die Anreicherung und Ausrichtung der strömenden magnetisch markierter Zellproben (90) korrigierbar ist.

7. Vorrichtung nach einem der vorstehenden Ansprüche, wobei die Länge der Anreicherungsstrecke (10) mindestens 15000 µm beträgt.

8. Vorrichtung nach einem der vorstehenden Ansprüche, wobei das Substrat (12) in seiner größten Ausdehnung höchstens 18000 µm misst.

9. Vorrichtung nach einem der vorstehenden Ansprüche, wobei der magnetoresistive Sensor (20) einen GMR-, TMR- oder AMR-Sensor aufweist.

10. Verfahren zur Herstellung einer Vorrichtung nach einem der Ansprüche 1 bis 9 mit den folgenden Schritten:
- Herstellung eines magnetoresistiven Sensors (20) auf einem Halbleitersubstrat (12),
- Aufbringung eines zweiten Abschnitts einer Anreicherungsstrecke (10) auf dem Halbleitersubstrat (12),
- Packaging des Halbleitersubstrats (12) auf einen Träger (13) und Herausführen elektrischer Kontakte (1-7, 18, 28) des magnetoresistiven Sensors (20) auf den Träger (13),
- Ausformung eines ersten Abschnitts der Anreicherungsstrecke (10).

11. Verfahren nach Anspruch 10 bei dem der Schritt des Packaging des Halbleitersubstrats (12) derart vorgenommen wird, dass aus dem Packaging-Material (16) ein Mikrofluidikkanal (50) ausgeformt wird.

12. Verfahren nach Anspruch 10 oder 11 bei dem in einem Schritt mittels Spritzgießen ein Mikrofluidikkanal (50) ausgeformt wird.

13. Verfahren nach einem der Ansprüche 10 bis 12 bei dem die magnetischen Führungsstreifen (15) des ersten Abschnitts der Anreicherungsstrecke (10) direkt auf den Kanalboden abgeschieden werden, insbesondere mittels thermischem Verdampfen oder Sputtern.

14. Verfahren nach einem der Ansprüche 10 bis 13 wobei die magnetischen Führungsstreifen (15) des zweiten Abschnitts der Anreicherungsstrecke direkt auf das Halbleitersubstrat (12) abgeschieden werden, insbesondere mittels thermischem Verdampfen oder Sputtern.

15. Verfahren zur magnetischen Zelldetektion bei dem eine magnetisch markierte Zellprobe (90) in eine Vorrichtung nach einem der Ansprüche 1 bis 9 injiziert wird.

## Claims

1. Device for magnetic flow cytometry, having
- a magnetoresistive sensor (20) on a substrate (12), and
- an enrichment path (10) for aligning and enriching magnetically marked cell samples (90), wherein
the enrichment path (10) comprises a first section and a second section, the second section being arranged on the substrate (12) and the first section being arranged next to the substrate (12) on a carrier (13), so that the enrichment path (10) extends over an edge of the substrate (12).

2. Device according to Claim 1, wherein the enrichment path (10) comprises magnetic guide strips (15).

3. Device according to Claim 2, wherein the magnetic guide strips (15) are ferromagnetic.

4. Device according to one of the preceding Claims 2 and 3, wherein a flow chamber (50), in particular a microfluidic channel, is formed along the enrichment path (10) in such a way that a magnetically marked cell sample (90) flowing through the microfluidic channel (50) is aligned on the magnetic guide strips (15).

5. Device according to one of the preceding claims, having a magnet, wherein the magnet is arranged in such a way that a magnetically marked cell sample (90) flowing through the microfluidic channel (50) is enriched on the channel bottom by the magnetic field of the magnet.

6. Device according to one of the preceding claims, wherein a first section and a second section of the microfluidic channel (50), as well as the magnetoresistive sensor (20), are arranged in such a way that a magnetically marked cell sample (90) flowing through the microfluidic channel (50) is guided first by the first section of the microfluidic channel (50) over the first section of the enrichment path (10), then by the second section of the microfluidic channel (50) over the second section of the enrichment path (10) and over the sensor (20), the second sections of the microfluidic channel (50) and of the enrichment path (10) being configured in such a way that, by these, an offset from the respective first sections of the microfluidic channel (50) and of the enrichment path (10) in relation to the enrichment and alignment of the flowing magnetically marked cell sample (90) can be corrected.

7. Device according to one of the preceding claims, wherein the length of the enrichment path (10) is at least 15000 µm.

8. Device according to one of the preceding claims, wherein the substrate (12) measures at most 18000 µm in its largest dimension.

9. Device according to one of the preceding claims, wherein the magnetoresistive sensor (20) comprises a GMR, TMR or AMR sensor.

10. Method for producing a device according to one of Claims 1 to 9, having the following steps:
- producing a magnetoresistive sensor (20) on a semiconductor substrate (12),
- applying a second section of an enrichment path (10) on the semiconductor substrate (12),
- packaging the semiconductor substrate (12) onto a carrier (13) and leading out the electrical contacts (1-7, 18, 28) of the magnetoresistive sensor (20) on the carrier (13),
- forming a first section of the enrichment path (10).

11. Method according to Claim 10, wherein the step of packaging the semiconductor substrate (12) is carried out in such a way that a microfluidic channel (50) is formed from the packaging material (16).

12. Method according to Claim 10 or 11, wherein a microfluidic channel (50) is formed in a step by means of injection molding.

13. Method according to one of Claims 10 to 12, wherein the magnetic guide strips (15) of the first section of the enrichment path (10) are deposited directly onto the channel bottom, in particular by means of thermal evaporation or sputtering.

14. Method according to one of Claims 10 to 13, wherein the magnetic guide strips (15) of the second section of the enrichment path are deposited directly onto the semiconductor substrate (12), in particular by means of thermal evaporation or sputtering.

15. Method for magnetic cell detection, wherein a magnetically marked cell sample (90) is injected into a device according to one of Claims 1 to 9.

## Revendications

1. Dispositif destiné à la cytométrie en flux magnétique comprenant
- un capteur magnétorésistif (20) sur un substrat (12) et
- une zone d'enrichissement (10) destinée à l'orientation et à l'enrichissement d'échantillons de cellules à marquage magnétique (90), la zone d'enrichissement (10) présentant une première et une deuxième section, la deuxième section étant disposée sur le substrat (12) et la première section étant disposée à côté du substrat (12) sur un support (13), de sorte que la zone d'enrichissement (10) s'étend pardessus un bord du substrat (12).

2. Dispositif selon la revendication 1, dans lequel la zone d'enrichissement (10) présente des languettes de guidage magnétiques (15).

3. Dispositif selon la revendication 2, dans lequel les languettes de guidage magnétiques (15) sont ferromagnétiques.

4. Dispositif selon l'une des revendications précédentes 2 ou 3, dans lequel une chambre de flux (50), en particulier un canal microfluidique, est configurée le long de la zone d'enrichissement (10) de sorte qu'un échantillon de cellules à marquage magnétique (90) circulant à travers le canal microfluidique (50) est orienté vers les languettes de guidage magnétiques (15).

5. Dispositif selon l'une des revendications précédentes comprenant un aimant, dans lequel l'aimant est disposé de sorte qu'un échantillon de cellules à marquage magnétique (90) circulant à travers le canal microfluidique (50) est enrichi par le biais du champ magnétique de l'aimant au fond du canal.

6. Dispositif selon l'une des revendications précédentes, dans lequel une première section et une deuxième section du canal microfluidique (50) ainsi que le capteur magnétorésistif (20) sont disposés de sorte qu'un échantillon de cellules à marquage magnétique (90) circulant à travers le canal microfluidique (50) est tout d'abord guidé à travers la première section du canal microfluidique (50) via la première section de la zone d'enrichissement (10), ensuite à travers la deuxième section du canal microfluidique (50) via la deuxième section de la zone d'enrichissement (10) et via le capteur (20), dans lequel les deuxièmes sections du canal microfluidique (50) et de la zone d'enrichissement (10) sont arrangées de sorte que par leur biais un décalage vis-à-vis respectivement des premières sections du canal microfluidique (50) et de la zone d'enrichissement (10) par rapport à l'enrichissement et à l'orientation des échantillons de cellules à marquage magnétique (90) en circulation peut être corrigé.

7. Dispositif selon l'une des revendications précédentes, dans lequel la longueur de la zone d'enrichissement (10) représente au moins 15000 µm.

8. Dispositif selon l'une des revendications précédentes, dans lequel le substrat (12) mesure dans son allongement maximal tout au plus 18000 µm.

9. Dispositif selon l'une des revendications précédentes, dans lequel le capteur magnétorésistif (20) présente un capteur GMR, TMR ou AMR.

10. Procédé destiné à la fabrication d'un dispositif selon l'une des revendications 1 à 9 comprenant les étapes suivantes :
- la fabrication d'un capteur magnétorésistif (20) sur un substrat semi-conducteur (12),
- la mise en place d'une deuxième section d'une zone d'enrichissement (10) sur le substrat semi-conducteur (12),
- le conditionnement du substrat semi-conducteur (12) sur un support (13) et la sortie de contact électriques (1-7, 18, 28) du capteur magnétorésistif (20) sur le support (13),
- la formation d'une première section de la zone d'enrichissement (10).

11. Procédé selon la revendication 10, dans lequel l'étape du conditionnement du substrat semi-conducteur (12) est entreprise de telle sorte qu'un canal microfluidique (50) est formé à partir du matériau de conditionnement (16).

12. Procédé selon la revendication 10 ou 11, dans lequel un canal microfluidique (50) est formé lors d'une étape au moyen d'un moulage par injection.

13. Procédé selon l'une des revendications 10 à 12, dans lequel les languettes de guidage magnétiques (15) de la première section de la zone d'enrichissement (10) sont isolées directement sur le fond du canal, en particulier au moyen d'une évaporation thermique ou d'une pulvérisation.

14. Procédé selon l'une des revendications 10 à 13, dans lequel les languettes de guidage magnétiques (15) de la deuxième section de la zone d'enrichissement sont isolées directement sur le substrat semi-conducteur (12), en particulier au moyen d'une évaporation thermique ou d'une pulvérisation.

15. Procédé destiné à la détection de cellules magnétiques, dans lequel un échantillon de cellules à marquage magnétique (90) est injecté dans un dispositif selon l'une des revendications 1 à 9.
